(19) Europäisches Patentamt
European Patent Office
Office européen des brevets

(11) Veröffentlichungsnummer: **0 133 692**
**A2**

(12) # EUROPÄISCHE PATENTANMELDUNG

(21) Anmeldenummer: **84109211.7**

(22) Anmeldetag: **03.08.84**

(51) Int. Cl.[4]: **C 07 C 103/365**
C 07 C 102/04

(30) Priorität: **16.08.83 DE 3329446**

(43) Veröffentlichungstag der Anmeldung:
**06.03.85 Patentblatt 85/10**

(84) Benannte Vertragsstaaten:
**CH DE FR GB IT LI**

(71) Anmelder: **BAYER AG**
**Konzernverwaltung RP Patentabteilung**
**D-5090 Leverkusen 1 Bayerwerk(DE)**

(72) Erfinder: **Kadelka, Jürgen, Dr.**
**Bodelschwinghstrasse 12**
**D-4150 Krefeld 1(DE)**

(72) Erfinder: **Schwarz, Hans-Helmut, Dr.**
**Ratherstrasse 90**
**D-4150 Krefeld 1(DE)**

(54) **Verfahren zur Herstellung unterschiedlich derivatisierter alpha, omega-Dicarbonsäuren.**

(57) Unterschiedlich derivatisierte α, ω-Dicarbonsäuren werden hergestellt durch Umsetzung ungesättigter Carbonsäurederivate mit Kohlenmonoxid und mit einer nukleophilen Komponente mit mindestens einem beweglichen Wasserstoffatom in Gegenwart von Kobaltverbindungen und in Gegenwart einer oder mehrerer tertiärer Stickstoffbasen bei erhöhtem Druck und erhöhter Temperatur.

EP 0 133 692 A2

Croydon Printing Company Ltd.

BAYER AKTIENGESELLSCHAFT 5090 Leverkusen, Bayerwerk

Konzernverwaltung RP
Patentabteilung Bg/Hed-c

# Verfahren zur Herstellung unterschiedlich derivatisierter α, ω-Dicarbonsäuren

Die Erfindung betrifft ein Verfahren zur Herstellung unterschiedlich derivatisierter α, ω-Dicarbonsäuren durch Umsetzung ungesättigter Carbonsäurederivate mit Kohlenmonoxid und mit einer nukleophilen Komponente mit mindestens einem beweglichen Wasserstoffatom in Gegenwart von Kobaltverbindungen und in Gegenwart von einer oder mehrerer tertiärer Stickstoffbasen bei erhöhtem Druck und erhöhter Temperatur.

Die Synthese unterschiedlich derivatisierter α, ω-Dicarbonsäuren der allgemeinen Formel

$$X^1\text{-CO-}(CH_2)_n\text{-CO-}X^2 \quad (X^1 \neq X^2;\ n > 1;\ \text{mit X z.B. für O Alkyl})$$

läßt sich auf verschiedenen Wegen durchführen. Ausgehend von einer α, ω-Dicarbonsäure liefert z.B. die gezielte Halbveresterung infolge der gleichen Reaktivität der

beiden Carboxylgruppen zwangsläufig auch den Diester, wodurch die Ausbeuten an erwünschtem Halbester niedrig sind (J. Chem. Soc. 1936, 902).

Dicarbonsäuremonoanilid-Derivate lassen sich durch Ammonolyse entsprechender N-substituierter cyclischer Imide gewinnen (s. CA. 68, 77 926 t). Diese Methode ist u.a. beschränkt durch die Zugänglichkeit der entsprechenden Imide.

Eine generelle Möglichkeit unterschiedlich derivatisierte $\alpha,\omega$-Dicarbonsäuren zu synthetisieren wird in Tetrahedron Letters 1977 (22), 1875 beschrieben. Dabei wird ein Molekülende einer $\alpha,\omega$-Dicarbonsäure durch Anbindung an eine heterogene Matrix blockiert und das freie Molekülende in der gewünschten Weise derivatisiert. Nach Abspaltung der so monoderivatisierten Dicarbonsäure von der Polymermatrix kann dann das andere Molekülende zusätzlich derivatisiert werden. Nachteilig bei dieser Methode sind die Vielzahl der Reaktionsschritte sowie die notwendige Verwendung aufwendiger Hilfssysteme.

Ferner ist bekannt, daß man durch Umsetzung von Olefinen mit Kohlenmonoxid und einer H-aciden Komponente, wie Wasser, Alkohol oder Amin, in Gegenwart eines Katalysators, der ein Metall der 8. Nebengruppe des Periodensystems der Elemente enthält, Carbonsäuren bzw. die entsprechenden Carbonsäurederivate herstellen kann (J. Falbe, Synthesen mit Kohlenmonoxid, Springer-Verlag, Berlin-Heidelberg-New York, 1967).

Bei Verwendung von Kobalt-haltigen Katalysatoren werden auch bei Einsatz von Olefinen mit innenständiger Doppelbindung überwiegend terminal funktionalisierte Produkte gebildet. Dieser Effekt wird verstärkt durch Zusatz von tertiären aromatischen Stickstoffbasen, wie Pyridin oder $\gamma$-Picolin (vgl. New Synthesis with Carbon Monoxide, Springer-Verlag, Berlin-Heidelberg-New York, 1980 und die DE-PS 30 23 765).

Es ist darüber hinaus bekannt, daß ungesättigte Carbonsäurealkylester mit innenständiger Doppelbindung sich in Gegenwart von Kobaltkatalysatoren und tertiären aromatischen Stickstoffbasen mit Kohlenmonoxid und einem Alkanol, das der Esterkomponente des Carbonsäurealkylesters entspricht, zu $\alpha,\omega$-Dicarbonsäuredialkylestern umsetzen lassen (vgl. DE-AS 27 13 195).

Weiterhin ist bekannt (s. EP-OS 80 957) gesättigte, lineare $\alpha,\omega$-Dicarbonsäurediester durch Umsetzung $\alpha$,ß-ungesättigter Carbonsäureester in Gegenwart von Kobaltkatalysatoren und tertiären aromatischen Stickstoffbasen mit Kohlenmonoxid und Alkohol herzustellen. Diese Methode ist jedoch auf die Herstellung von gesättigten, linearen $\alpha,\omega$-Dicarbonsäurediestern beschränkt.

Der Erfindung lag daher die Aufgabe zugrunde, eine allgemein anwendbare, möglichst einfache, d.h. möglichst wenige Reaktionsstufen umfassende, wirtschaftlich akzeptable Methode zur Synthese unterschiedlich derivatisierter $\alpha,\omega$-Dicarbonsäuren zu finden, wie den $\alpha,\omega$-Dicarbonsäuredithioestern oder den eine Ester- und eine Thioestergruppe, eine Ester- und eine Amidgruppe sowie den eine Thioester- und eine Amidgruppe enthaltenden $\alpha,\omega$-Dicarbonsäurederivaten.

Es wurde nun gefunden, daß man unterschiedlich derivatisierte $\alpha,\omega$-Dicarbonsäuren durch Umsetzung ungesättigter Carbonsäurederivate der Formel

$$R\text{-}CO\text{-}X^1 \qquad (I),$$

worin

R einen olefinisch ungesättigten, unverzweigten Alkylrest mit 2 bis 30 Kohlenstoffatomen darstellt und

$X^1$ für $-OR^1$ und $-SR^1$ steht, wobei $R^1$ einen Alkyl-, Cycloalkyl- oder Aralkylrest mit 1 bis 20 Kohlenstoffatomen oder einen Arylrest mit 6 bis 20 Kohlenstoffatomen darstellt, wobei gegebenenfalls der jeweilige Alkyl-, Cycloalkyl-, Aralkyl- oder Arylrest ein- oder mehrfach durch eine Alkyl- und/oder Alkoxygruppe mit 1 bis 12 Kohlenstoffatomen und/oder durch Fluor, Chlor, Brom und/oder Jod und/oder durch eine durch Fluor, Chlor, Brom und/oder Jod ein- oder mehrfach substituierte Alkyl- und/oder Alkoxygruppe mit 1 bis 12 Kohlenstoffatomen substituiert ist,

mit Kohlenmonoxid und mit einer H-aciden nukleophilen Verbindung in Gegenwart von Kobaltverbindungen und in Gegenwart einer oder mehreren tertiären Stickstoffbasen bei erhöhtem Druck und erhöhter Temperatur erhält, wenn man als H-acide nukleophile Verbindung eine solche der Formel

$$HX^2 \qquad (II),$$

worin

$X^2$ für $-SR^2$, $-NH_2$, $-NHR^2$ und $-NR^2R^3$ steht, wobei $R^2$ und $R^3$ gleich oder verschieden sind und die für $R^1$ angegebene Bedeutung haben,

einsetzt mit der Maßgabe, daß $X^1$ und $X^2$ stets einen unterschiedlichen Rest am Schwefelatom tragen, wenn $X^1$ und $X^2$ für $-SR^1$ und $-SR^2$ stehen.

Als olefinisch ungesättigte, unverzweigte Alkylreste R kommen insbesondere solche mit 2 bis 18 Kohlenstoffatomen in Betracht, wie Ethenyl, Prop-1-en-1-yl, Prop-2-en-1-yl, But-1-en-1-yl, But-2-en-1-yl, But-3-en-1-yl, Pent-1-en-1-yl, Pent-2-en-1-yl, Pent-3-en-1-yl, Pent-4-en-1-yl, Hex-1-en-1-yl, Hept-1-en-1-yl, Oct-1-en-1-yl, Non-1-en-1-yl, Dec-1-en-1-yl, Dec-9-en-1-yl, und Heptadec-8-en-1-yl, bevorzugt Ethenyl, Prop-1-en-1-yl, But-1-en-1-yl, Dec-1-en-1-yl, Dec-9-en-1-yl und Heptadec-8-en-1-yl.

Als Alkylreste $R^1$ der entsprechenden Alkohol- oder Thioalkoholgruppe $X^1$ der Formel (I) kommen vorzugsweise solche mit 1 bis 12 Kohlenstoffatomen in Betracht, wie der

Methyl-, Ethyl-, n-Propyl-, iso-Propyl-, n-Butyl-, 2-Ethylhexyl-, n-Heptyl- und der Decylrest, bevorzugt der Methyl-, Ethyl-, n-Propyl- und n-Butylrest, als Cycloalkylreste solche mit 6 bis 12 Kohlenstoffatomen, wie der Cyclohexyl-, Cyclooctyl- und der Cyclododecylrest, bevorzugt der Cyclohexylrest, als Aralkylreste solche mit 7 bis 12 Kohlenstoffatomen, wie der Benzyl-, 1-Phenyl-eth-1-yl- und der 2-Phenyl-eth-1-ylrest, bevorzugt der Benzylrest und als Arylreste solche mit 6 bis 10 Kohlenstoffatomen, wie der Phenyl- oder der Naphthylrest, bevorzugt der Phenylrest.

Die Alkyl-, Cycloalkyl-, Aralkyl- und Arylreste können noch zusätzlich durch eine oder mehrere Alkyl- und/oder Alkoxygruppen mit 1 bis 12 Kohlenstoffatomen und/oder durch Fluor, Chlor, Brom und/oder Iod, bevorzugt durch Fluor oder Chlor substituiert sein. Zum Beispiel seien als mögliche Reste mit solchen Substituenten genannt: o-, m-, p-Kresyl, o-, m-, p-Chlorphenyl, o-, m-, p-Fluorphenyl, 3,5-Dichlorphenyl und Pentafluorphenyl.

In das erfindungsgemäße Verfahren können demnach als ungesättigte Carbonsäurederivate zum Beispiel eingesetzt werden Acrylsäure-, Crotonsäure-, Pent-3-ensäure-, Pent-4-en-säure-, Undecylensäure-, Ölsäuremethylester, -thiomethylester, -phenylester und -thiophenylester.

Als H-acide nukleophile Verbindungen der Formel (II) können in das erfindungsgemäße Verfahren eingesetzt werden aliphatische und aromatische Thioalkohole, Ammoniak sowie aliphatische und aromatische Amine. Welcher

Thioalkohol oder welches Amin als H-acide nukleophile Komponente in das erfindungsgemäße Verfahren eingesetzt wird, hängt davon ab, welche unterschiedlich derivatisierte $\alpha$, $\omega$ -Dicarbonsäure hergestellt werden soll. So können als aliphatische oder aromatische Thioalkohole eingesetzt werden Methyl-, Ethyl-, n-Propyl-, n-Butyl- oder Benzylmercaptan, Thiophenol oder substituierte Thioalkohole, wie p-Thiokresol, bevorzugt Methyl-, Ethyl- und Benzylmercaptan sowie Thiophenol. Neben Ammoniak können als aliphatische und aromatische Amine mit mindestens einem beweglichen Wasserstoffatom am Stickstoff eingesetzt werden Methyl-, Ethyl-, Propyl-, Butyl-, Benzyl-, Dimethyl-, Diethyl-, Dipropyl-, Dibutyl-, Cyclohexyl-, N-Methylcyclohexyl-, N-Ethylcyclohexylamin oder Anilin oder substituierte Aniline, wie N-Methyl-, N-Ethyl-, o-, m-, p-Chlor-, o-, m-, p-Fluor-, o-, m-, p-Methyl-, 2,3-, 2,4-, 2,6-, 3,4-, 3,5-Dichlor-, 3-Trifluormethyl-, 3,5-Bis (trifluormethyl)-, 2,4,5-, 2,4,6-Trichloranilin, bevorzugt Anilin und o-, m-, p-Fluoranilin; ebenso ist die Verwendung von Diaminen und höheren Aminen mit mindestens einem beweglichen Wasserstoffatom am Stickstoff, wie Ethylendiamin oder Hexamethylendiamin, möglich.

Der Umsatz der als Substrate eingesetzten ungesättigten Carbonsäurederivate der Formel (I) liegt in der Regel bei etwa 60 bis 100 %. Andererseits ist es für das erfindungsgemäße Verfahren unerheblich, die Umsätze des Substrates auf Werte unter 60 % zu begrenzen.

Ferner kann es zweckmäßig sein, die Umsetzung in Gegenwart eines inerten organischen Lösungs- und/oder Verdünnungsmittels durchzuführen. Beispielsweise kann man die erfindungsgemäße Umsetzung in Gegenwart von Toluol, Tetrahydrofuran, Dioxan, Acetonitril und/oder Di-n-butylether durchführen. Die Menge des einzusetzenden inerten Lösungs- und/oder Verdünnungsmittels ist nicht kritisch und kann erforderlichenfalls durch einige Vorversuche leicht ermittelt werden.

Die einzusetzende Menge an H-acider nukleophiler Verbindung der allgemeinen Formel (II) sollte zumindestens äquimolar bezogen auf das umzusetzende ungesättigte Carbonsäurederivat sein. Als günstig haben sich Überschüsse an nukleophiler Komponente von etwa 1,1 bis 5 Mol/Mol ungesättigtes Carbonsäurederivat erwiesen. Andererseits behindern größere Überschüsse das erfindungsgemäße Verfahren nicht, da zudem die nukleophile Komponente bei manchen Umsetzungen als Lösungs- und/oder Verdünnungsmittel Verwendung finden kann, wodurch auf den Einsatz eines systemfremden Lösungs- und/oder Verdünnungsmittels verzichtet werden kann.

Für die Herstellung der unterschiedlich derivatisierten $\alpha,\omega$-Dicarbonsäurederivate werden Kohlenmonoxid-Drucke von etwa 50 bis 4000 bar angewandt. Als vorteilhaft haben sich Kohlenmonoxid-Drucke von 80 bis 1000, als besonders vorteilhaft solche von 100 bis 300 bar erwiesen.

Nach dem erfindungsgemäßen Verfahren ist es zweckmäßig, das Kohlenmonoxid im Überschuß, vornehmlich in 2 bis 100 molarem Überschuß, bezogen auf das umzusetzende Substrat, einzusetzen. Überschüssiges Kohlenmonoxid kann nach der Umsetzung leicht, z.B. durch Entspannung, von den übrigen Reaktionskomponenten abgetrennt und als solches wiederum verwendet werden.

Zur Erzielung akzeptabler Reaktionsgeschwindigkeiten ist es nützlich, dem Kohlenmonoxid etwa 0,5 bis 10 Vol.-% Wasserstoff, bevorzugt 1 bis 3 Vol.-% Wasserstoff beizumischen.

Bei den als Katalysatoren eingesetzten Kobaltverbindungen handelt es sich um Carbonylkomplexe bzw. Hydrocarbonylkomplexe des Kobalts. Es können jedoch auch Chloride, Bromide, Jodide, Acetate, Oxide, Carbonate, Sulfate oder sonstige Kobaltverbindungen, aus welchen unter den gegebenen Reaktionsbedingungen katalytisch aktive Kobaltcarbonylverbindungen entstehen, verwendet werden. Andererseits ist es auch möglich, bei Einsatz von nicht-carbonylhaltigen Kobaltverbindungen einen sogenannten Präformierschritt, d.h. eine gezielte Vorbehandlung der Kobaltverbindung zur Erzeugung der katalytisch aktiven Verbindung, der eigentlichen Umsetzung voranzustellen. Bevorzugt eingesetzte Kobaltverbindung ist das Dikobaltoctacarbonyl, $Co_2(CO)_8$. Es ist zweckmäßig, die Kobaltcarbonyl-Komplexe in solchen Mengen einzusetzen, daß pro g-Atom Kobalt etwa

1 bis 200 Mol Substrat, bevorzugt 10 bis 100 Mol Substrat vorliegen. Besonders vorteilhaft sind Substrat : Kobalt-Verhältnisse von 15 bis 75:1.

Das erfindungsgemäße Verfahren wird in Gegenwart von tertiären Stickstoffbasen, vorzugsweise aromatischen tertiären Stickstoffbasen, durchgeführt, deren $pK_A$-Wert bei etwa 3 bis 10, bevorzugt bei etwa 4 bis 9 liegt.

Als Stickstoffbasen kommen insbesondere N-heterocyclische Stickstoffverbindungen, die in ortho-Stellung zum Heteroatom nicht substituiert sind, in Betracht. Beispielsweise werden eingesetzt: Pyridin, Isochinolin, ß-Picolin, γ-Picolin, 3,5-Lutidin, 4-Ethylpyridin und/oder 4-Benzylpyridin, bevorzugt Pyridin, γ-Picolin und/oder Isochinolin.

Die einzusetzenden Basen können neben Stickstoffatomen auch andere Heteroatome, z.B. Sauerstoff oder Chlor, enthalten.

Üblicherweise werden die Stickstoffbasen, die sowohl einzeln als auch im Gemisch untereinander eingesetzt werden können, und die ungesättigten Carbonsäurederivate, das Substrat, in einem Molverhältnis von etwa 0,01:1 bis 2:1, bevorzugt in einem Molverhältnis von 0,04:1 bis 0,8:1. Größere Basenüberschüsse bringen wirtschaftlich keine Vorteile.

Die Reaktion wird bei einer Temperatur von etwa 90 bis 220°C, vorzugsweise von 110 bis 180°C durchgeführt. Je nachdem, welche gemischt derivatisierte $\alpha,\omega$-Dicarbonsäure synthetisiert werden soll, ist die Festlegung einer oberen Reaktionstemperatur sinnvoll, um Folgereaktion der $\alpha,\omega$-Dicarbonsäurederivate, z.B. Ringschlußreaktionen, zu verhindern. Die für die jeweilige Umsetzung optimale Reaktionstemperatur läßt sich in einigen Vorversuchen leicht ermitteln.

Die Durchführung der Reaktion kann sowohl diskontinuierlich als auch kontinuierlich erfolgen.

Das erfindungsgemäße Verfahren sei am Beispiel der Umsetzung von Crotonsäuremethylester mit 3,5-Dichloranilin und Kohlenmonoxid wie folgt formelmäßig dargestellt:

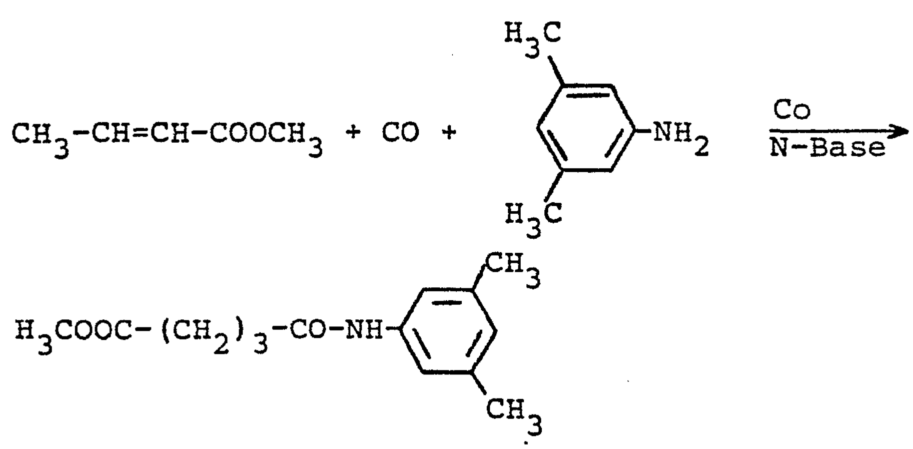

Das erfindungsgemäße Verfahren kann allgemein wie folgt durchgeführt werden:
Ein mit Stickstoff oder Argon gespülter Autoklav wird z.B. mit dem Ammoniak, Thioalkohol oder dem Amin, der oder den tert.-Stickstoffbase(n), dem Kobaltkatalysator, dem ungesättigten Carbonsäurederivat und gegebenenfalls dem inerten Lösungs- und/oder Verdünnungsmittel beschickt. Dann wird bei Raumtemperatur Kohlenmonoxid, welches die notwendige Menge Wasserstoff enthält, aufgepreßt, und zwar soviel, daß bei der gewünschten Reaktionstemperatur der vorgegebene Reaktionsdruck aufgebaut wird. Anschließend wird der Autoklaveninhalt auf Reaktionstemperatur aufgeheizt und unter Rühren die vorgegebene Zeit bei dieser Temperatur (± 3°C) gehalten. Der Reaktionsdruck wird während der Reaktion durch sukzessive Nachspeisung des Reaktionsgases in einem Bereich von ± 5 bar konstant gehalten. Abschließend wird das Produktgemisch abgekühlt, der Autoklav entspannt und das erhaltene Reaktionsgemisch, je nach Erfordernis nach Zusatz eines Lösungsmittels, gaschromatographisch analysiert. Das Produktgemisch kann durch Destillation bzw. Kristallisation in jeweils üblicher Weise aufgearbeitet werden.

Nach dem erfindungsgemäßen Verfahren lassen sich z.B. α,ω-Dicarbonsäurealkylester-thioalkylester, α,ω-Dicarbonsäurealkylester-thioarylester, α,ω-Dicarbonsäurearylester-thioalkylester, α,ω-Dicarbonsäurearylester-thioarylester, α,ω-Dicarbonsäureamid-alkylester, α,ω-Dicarbonsäureamid-arylester,

α, ω-Dicarbonsäure-amid-thioalkylester, α, ω-Dicarbonsäureamid-thioarylester, α, ω-Dicarbonsäure-thioalkylester-thioalkylester (mit unterschiedlichen Thioalkylresten), α, ω-Dicarbonsäure-thioarylesterthioarylester (mit unterschiedlichen Thioarylresten) und α, ω-Dicarbonsäure-thioalkylester-thioarylester in einem Reaktionsschritt synthetisieren.

Die durch Umsetzung von Acrylsäuremethylester mit Kohlenmonoxid und 3,5-Dichlor- oder 3,5-Bis(trifluormethyl)anilin erhaltenen Bernsteinsäure-3,5-dichloranilid-methylester und Bernsteinsäure-3,5-bis(trifluormethyl)anilid-methylester sowie die durch Umsetzung von Crotonsäuremethylester mit Kohlenmonoxid und 3,5-Dichlor- oder 3,5-Dimethylanilin erhaltenen Glutarsäure-3,5-dichloranilid-methylester und Glutarsäure-3,5-dimethylanilid-methylester sind neue Verbindungen. Die Charakterisierung der neuen Ester erfolgte durch Protonen-Kernresonanz-Spektrometrie.

Es ist besonders überraschend, daß nach dem erfindungsgemäßen Verfahren α, ω-Dicarbonsäureester-amide in solch hohen Ausbeuten erhalten werden, da es z.B. aus "Synthesen mit Kohlenmonoxid", Seite 16, Springer-Verlag, Berlin-Heidelberg-New York, 1967 bekannt ist, daß große Überschüsse an Aminen bezogen auf den Kobaltkatalysator - dies ist beim erfindungsgemäßen Verfahren ja der Fall - die Hydrocarbonylierungsreaktionen hemmen.

Darüber hinaus ist zusätzlich bekannt, daß Carbonsäureester häufig bereits bei Raumtemperatur mit Aminen zu den entsprechenden Carbonsäureamiden reagieren (vgl.

Houben-Weyl, "Methoden der organischen Chemie", Band XI/2, S. 20 ff, Georg-Thieme Verlag Stuttgart, 1958). Es war daher nicht zu erwarten, daß die Esterfunktionen der eingesetzten Substrate unter den erfindungsgemäßen Reaktionsbedingungen praktisch nicht aminolysiert werden.

Ebenso überraschend sind die guten Ausbeuten an $\alpha,\omega$-Dicarbonsäureester-thioestern, die nach den erfindungsgemäßen Verfahren erhalten werden. Aus H.R. Christen "Grundlagen der organischen Chemie", Tabelle 11.2., Seite 482, Verlag Sauerländer, Aarau, 1970 ist nämlich bekannt, daß Carbonsäureester durch Thioalkohole nukleophil substituiert werden, da die Alkoxygruppe im Vergleich zur Thioxygruppe die bessere Abgangsgruppe darstellt. In der gleichen Literaturstelle auf Seite 383 wird zusätzlich beschrieben, daß Thioxyreste starke nukleophile Reagenzien sind, wodurch bei der Umsetzung eine Verbindung mit aktivierter Doppelbindung mit einem Thioalkohol die überwiegende Addition des Thioalkohols an die Doppelbindung anstelle einer Carbonylierungsreaktion zu erwarten wäre.

Die nach dem erfindungsgemäßen Verfahren erhaltenen unterschiedlich derivatisierten $\alpha,\omega$-Dicarbonsäuren eignen sich als Additive bei der Kautschuk-Herstellung sowie als Antioxidantien. Desweiteren können sie als Ausgangsstoffe für die Synthese von Herbiziden, Fungiziden, Insektiziden und von Akariziden sowie von physiologischen Wirkstoffen dienen.

Beispiel 1

In einem mit Stickstoff gespülten 0,25 l-Schüttelautoklaven wurden 50,1 g Crotonsäuremethylester, 69,8 g Anilin, 7,9 g Pyridin und 3,42 g $Co_2(CO)_8$ vorgelegt.

Nach dem Verschließen des Autoklavens wurde so viel Kohlenmonoxid, welches ca. 2 Vol.-% Wasserstoff enthielt, aufgepreßt, daß bei Erreichen der Reaktionstemperatur der Gesamtgasdruck 150 bar betrug. Anschließend wurde der Autoklav mittels einer elektrischen Heizung auf 170°C aufgeheizt und 2 Stunden unter Fortsetzung der Schüttelung bei dieser Temperatur gehalten. Verbrauchtes Reaktionsgas wurde durch sukzessive Nachspeisung von frischem Reaktionsgas ersetzt, so daß der Reaktionsdruck bei 150 bar (± 5 bar) konstant gehalten wurde. Nach dem Abkühlen des Reaktionsgemisches wurde der Autoklav entspannt und das Produktgemisch gaschromatographisch analysiert.
Die Analyse zeigt, daß bezogen auf einen Umsatz an Crotonsäuremethylester von 81,4 Mol-% 51,5 Mol-% gemischt derivatisierte $C_5$-Dicarbonsäurederivate entstanden waren. Der Anteil an $\alpha$, $\omega$ Produkt (Glutarsäureanilidmethylester) betrug 98,3 %.

Beispiel 2

Beispiel 1 wurde wiederholt mit den Ausnahmen, daß 58,2 g Anilin eingesetzt und die Reaktion 4 Stunden

bei 150°C durchgeführt wurden. Bezogen auf einen 52,9 mol.-%igen Umsatz waren 54,5 Mol-% gemischt derivatisierte $C_5$-Dicarbonsäurederivate entstanden; die Linearität betrug 93,4 %.

Beispiel 3

Analog Beispiel 1 wurden 40,1 g Crotonsäuremethylester, 72,7 g 3,5-Dimethylanilin, 6,33 g Pyridin und 2,74 g $Co_2(CO)_8$ mit Kohlenmonoxid (+ 2 % $H_2$) umgesetzt. Nach 1,5-stündiger Reaktionszeit bei 170°C hatten sich 95,7 Mol-% des ungesättigten Esters umgesetzt; darauf bezogen betrug die Ausbeute an Glutarsäure-3,5-dimethylanilid-methylester 60,7 Mol.-% (Linearität ca. 90 %).

Glutarsäure-3,5-dimethylanilid-methylester

F = 116°C (aus Ethanol)

$^1$H-NMR: $\delta$= 1,98 ppm (m, 2H); $\delta$= 2,25 ppm (s, 6H);
$\delta$= 2,39 ppm (tr, 4H); $\delta$= 3,64 ppm (s, 3H);
$\delta$= 6,66 ppm (tr, 1H); $\delta$= 7,21 ppm (br, 2H);
$\delta$= 9,36 ppm (Offset, br, 1H).

Beispiel 4

Analog Beispiel 1 wurden 30,0 g Crotonsäuremethylester, 72,9 g 3,5-Dichloranilin, 4,75 g Pyridin, 10,8 g Tetrahydrofuran und 2,05 g $Co_2(CO)_8$ eingesetzt. Nach 1-stün-

diger Umsetzung unter 150 bar CO (+ 2 % $H_2$) und 170°C hatten sich 96,0 Mol-% des Crotonsäureesters umgesetzt; darauf bezogen waren 59,0 Mol-% gemischt derivatisierte $C_5$-Dicarbonsäurederivate entstanden, wovon 82,9 % Glutarsäure-3,5-dichloranilid-methylester waren.

Glutarsäure-3,5-dichloranilid-methylester

F = 160°C (aus Ethanol)

$^1$H-NMR: $\delta$ = 1,99 ppm (m, 2H); $\delta$ = 2,41 ppm (tr, 4H); $\delta$ = 3,65 ppm (s, 3H); $\delta$ = 7,00 ppm (tr, 1H); $\delta$ = 7,64 ppm (d, 2H); $\delta$ = 9,88 ppm (Offset, br, 1H).

Beispiel 5

Analog Beispiel 1 wurden 20,0 g Crotonsäuremethylester, 38,9 g 3,5-Dichloranilin, 3,16 g Pyridin, 43,3 g Tetrahydrofuran und 1,37 g $Co_2(CO)_8$ eingesetzt. Nach einer Reaktionszeit von 1 h bei 150°C hatten sich 87,8 Mol-% des Crotonsäureesters umgesetzt; darauf bezogen waren 62,5 Mol-% gemischt derivatisierte $C_5$-Dicarbonsäurederivate entstanden, die Linearität betrug 81,2 %.

Beispiel 6

Analog Beispiel 1 wurden 25,8 g Acrylsäuremethylester, 63,2 g 3,5-Dichloranilin, 4,75 g Pyridin, 21,6 g Tetrahydrofuran und 2,05 g $Co_2(CO)_8$ eingesetzt. Nach 1-stün-

diger Umsetzung bei 150°C hatte sich der Acrylester vollständig umgesetzt. Es hatten sich 57,2 g Bernsteinsäure-3,5-dichloranilid-methylester gebildet; dies entspricht einer Ausbeute von 69,3 Mol-%.

Bernsteinsäure-3,5-dichloranilid-methylester

F = 163°C (aus Ethanol)
$^1$H-NMR: $\delta$ = 2,67 ppm (s, 4H); $\delta$ = 3,67 ppm (s, 3H);
$\delta$ = 7,01 ppm (tr, 1H); $\delta$ = 7,63 ppm (d, 2H);
$\delta$ = 10,09 ppm (Offset, br, 1H).

Beispiel 7

Analog Beispiel 1 wurden 36,2 g Pent-3-ensäuremethylester, 55,9 g Anilin, 4,75 g Pyridin und 2,05 g $Co_2(CO)_8$ eingesetzt. Die Umsetzung erfolgte 2 Stunden bei 170°C. Die Analyse zeigte, daß sich 75,5 Mol-% Pentensäuremethylester umgesetzt hatten; darauf bezogen waren 59,3 Mol-% gemischt funktionalisierte $C_6$-Dicarbonsäurederivate entstanden, wovon 79,0 % Adipinsäureanilidmethylester waren.

Beispiel 8

Analog Beispiel 1 wurden 22,8 g Pent-3-ensäuremethylester, 44,1 g Thiophenol, 3,16 g Pyridin und 1,37 g $Co_2(CO)_8$ eingesetzt. Die Umsetzung wurde 1,5 Stunden bei 170°C durchgeführt. Bezogen auf einen Pentensäure-

methylester-Umsatz von 18,1 Mol-% hatten sich 28,2 Mol-% gemischt derivatisierte $C_6$-Dicarbonsäurederivate mit einem linearen Anteil an Adipinsäuremethylesterthiophenylester von 37,3 % gebildet.

Beispiel 9

Analog Beispiel 1 wurden 8,6 g Acrylsäuremethylester, 27,5 g 3,5-Bis(trifluormethyl)anilin, 1,58 g Pyridin, 28,8 g Tetrahydrofuran und 0,68 g $Co_2(CO)_8$ eingesetzt. Nach 0,5-stündiger Umsetzung bei 150°C hatten sich 93,0 Mol-% des Acrylesters umgesetzt. Darauf bezogen hatten sich 83,5 Mol-% gemischt funktionalisierte $C_4$-Dicarbonsäurederivate gebildet, woran der Anteil des Bernsteinsäure-3,5-bis(trifluormethyl)anilid-methylesters 92,6 % betrug.

Bernsteinsäure-3,5-bis(trifluormethyl)anilid-methylester

F = 93°C (aus Ether/Petrolether 1:1)

$^1$H-NMR: $\delta$ = 2,80 ppm (s,4H); $\delta$ = 3,78 ppm (s,3H); $\delta$ = 7,54 ppm (s,1H); $\delta$ = 8,01 ppm (s,2H); $\delta$ = 8,68 ppm (s,1H);

Beispiel 10

Analog Beispiel 1 wurden 8,6 g Acrylsäuremethylester, 27,5 g 3,5-Bis(trifluormethyl)anilin, 0,95 g Pyridin, 41,0 g Acetonitril und 0,68 g $Co_2(CO)_8$ 45 min bei 135°C

unter 150 bar CO (+ ca. 2 Vol.-% $H_2$) umgesetzt. Bei einem Acrylester-Umsatz von 98,3 Mol-% hatten sich mit einer Selektivität von 63,3 Mol-% gemischt funktionalisierte $C_4$-Dicarbonsäurederivate gebildet; der Anteil des linearen Bernsteinsäurederivates daran betrug 96,1 %.

Beispiel 11

Analog Beispiel 1 wurden 8,6 g Acrylsäuremethylester, 27,5 g 3,5-Bis(trifluormethyl)anilin, 0,32 g Pyridin, 14,4 g Tetrahydrofuran und 0,68 g $Co_2(CO)_8$ 30 min bei 135°C zur Reaktion gebracht. 98,4 Mol-% des Acrylsäuremethylesters hatten sich umgesetzt, wobei sich 95,5 Mol-% gemischt funktionalisierte $C_4$-Dicarbonsäurederivate gebildet hatten. Die Linearität betrug 95,9 %.

Beispiel 12

Beispiel 9 wurde wiederholt mit der Ausnahme, daß die Reaktionstemperatur 120°C betrug. Nach 30 min wurde ein Acrylesterumsatz von 92,9 Mol-% erzielt. Darauf bezogen hatten sich 86,4 Mol-% gemischt funktionalisierte $C_4$-Dicarbonsäurederivate gebildet; die Linearität lag bei 90,5 %.

Beispiel 13

Analog Beispiel 1 wurden in einem 0,5 l-Schüttelautoklaven 25,8 g Acrylsäuremethylester, 58,0 g 3-Trifluormethylanilin, 4,75 g Pyridin, 64,8 g Tetrahydrofuran

und 2,05 g $Co_2(CO)_8$ 45 min bei 135°C unter 150 bar CO (+ ca. 2 Vol.-% $H_2$) umgesetzt. Bezogen auf einen Acrylsäuremethylester-Umsatz von 99,1 Mol-% hatten sich 94,3 Mol-% gemischt funktionalisierte $C_4$-Dicarbonsäurederivate gebildet, wovon 96,6 % Bernsteinsäure-3-trifluormethylanilid-methylester waren.

Patentansprüche

1. Verfahren zur Herstellung unterschiedlich derivatiserter $\alpha$, $\omega$-Dicarbonsäuren durch Umsetzung ungesättigter Carbonsäurederivate der Formel

$$R\text{-}COX^1 ,$$

worin

R einen olefinisch ungesättigten, unverzweigten Alkylrest mit 2 bis 30 Kohlenstoffatomen darstellt und

$X^1$ für $-OR^1$ und $-SR^1$ steht, wobei $R^1$ einen Alkyl-, Cycloalkyl- oder Aralkylrest mit 1 bis 20 Kohlenstoffatomen oder einen Arylrest mit 6 bis 20 Kohlenstoffatomen darstellt, wobei gegebenenfalls der jeweilige Alkyl-, Cycloalkyl-, Aralkyl- oder Arylrest ein- oder mehrfach durch eine Alkyl- und/oder Alkoxygruppe mit 1 bis 12 Kohlenstoffatomen und/oder durch Fluor, Chlor, Brom und/oder Jod und/oder durch eine durch Fluor, Chlor, Brom und/oder Jod ein- oder mehrfach substituierte Alkyl- und/oder Alkoxygruppe mit 1 bis 12 Kohlenstoffatomen substituiert ist,

mit Kohlenmonoxid und mit einer H-aciden nukleophilen Verbindung in Gegenwart von Kobaltverbindungen und in Gegenwart von einer oder mehreren tertiären Stickstoffbasen bei erhöhtem Druck und erhöhter Temperatur, dadurch gekennzeichnet, daß

man als H-acide nukleophile Verbindung eine solche der Formel

$$HX^2 \quad ,$$

worin

$X^2$ für $-SR^2$, $-NH_2$, $-NHR^2$ und $-NR^2R^3$ steht, wobei $R^2$ und $R^3$ gleich oder verschieden sind und die für $R^1$ angegebene Bedeutung haben,

einsetzt mit der Maßgabe, daß $X^1$ und $X^2$ stets einen unterschiedlichen Rest am Schwefelatom tragen, wenn $X^1$ und $X^2$ für $-SR^1$ und $-SR^2$ stehen.

2. Verfahren nach Anspruch 1, dadurch gekennzeichnet, daß man als H-acide nukleophile Verbindungen Methylmercaptan, Ethylmercaptan, n-Propylmercaptan, n-Butylmercaptan, Benzylmercaptan, Thiophenol, p-Thiokresol, Ammoniak, Methylamin, Ethylamin, Propylamin, Butylamin, Benzylamin, Dimethylamin, Diethylamin, Dipropylamin, Dibutylamin, Cyclohexylamin, N-Methylcyclohexylamin, N-Ethylcyclohexylamin, Anilin, N-Methylanilin, N-Ethylanilin, o-, m-, p-Chloranilin, o-, m-, p-Fluoroanilin, o-, m-, p-Methylanilin, 2,3-, 2,4-, 2,6-, 3,4-, 3,5-Dichloranilin, 3-Trifluormethyl-, 3,5-Bis(trifluormethyl)anilin, 2,4,5-, 2,4,6-Trichloranilin, Ethylendiamin oder Hexamethylendiamin einsetzt.

3. Verfahren nach Ansprüchen 1 und 2, dadurch gekennzeichnet, daß man als H-acide nukleophile Verbindungen Methylmercaptan, Ethylmercaptan, Benzylmercaptan, Thiophenol, Ammoniak, Anilin, o-Fluoranilin, m-Fluoranilin, p-Fluoranilin, 3,5-Dichloranilin, 3-Trifluormethylanilin oder 3,5-Bis(trifluormethyl)anilin einsetzt.

4. Verfahren nach Ansprüchen 1 bis 3, dadurch gekennzeichnet, daß man die H-acide nukleophile Verbindungen in mindestens äquimolaren Mengen bezogen auf das umzusetzende ungesättigte Carbonsäurederivat einsetzt.

5. Verfahren nach Ansprüchen 1 bis 4, dadurch gekennzeichnet, daß man die H-acide nukleophile Verbindungen in einer Menge von 1,1 bis 5 Mol pro Mol umzusetzendes ungesättigtes Carbonsäurederivat einsetzt.